# DEMANDE DE BREVET EUROPEEN

(11) **EP 2 949 593 A1**
(43) Date de publication de la demande: **02.12.2015**
(21) Numéro de dépôt: 15169113.6
(22) Date de dépôt: 26.05.2015
(51) Int. Cl.: B65D 47/18, B65D 47/20

(54) **APPAREIL DE DISTRIBUTION DE GOUTTES AMÉLIORÉ**

(30) Priorité: 26.05.2014 FR 1454738
(71) Demandeur: Techniplast, 27400 Louviers (FR)
(72) Inventeur: Lamboux, Jean-Philippe, 27370 Saint Didier des Bois (FR); Poullain, Yvan, 27400 Pintherville (FR)
(74) Mandataire: Petit, Maxime

(57) **Abrégé**

L'invention concerne un appareil de distribution de gouttes d'un produit liquide ou semi-liquide, comprenant :
-un tube (12) qui comprend, d'une part, une enveloppe externe déformable définissant un espace interne (16) renfermant le produit et, d'autre part, une ouverture,
-un embout de génération de gouttes (14) qui est disposé au niveau de l'ouverture et qui est apte, d'une part, à former et à expulser une goutte de produit de l'embout par suite d'une pression exercée sur l'enveloppe externe et, d'autre part, à empêcher tout retour de produit dans l'embout lorsque la pression sur l'enveloppe cesse.

## Description

L'invention concerne un appareil de distribution d'un produit liquide ou semi-liquide sous la forme de gouttes.

Les appareils connus à ce jour pour générer des gouttes de produit se rencontrent par exemple dans le domaine des produits ophtalmiques et se répartissent en deux catégories.

Dans la première catégorie on trouve les appareils monodose dont le contenant, en plastique souple (les contenants sont généralement fabriqués et proposés à la vente sous la forme d'un emballage renfermant une barrette de contenants fixés les uns aux autres de manière détachable par un utilisateur), est scellé par un bouchon qu'il convient de détacher de manière irréversible avant toute utilisation. L'utilisateur presse fortement sur l'enveloppe externe du contenant afin de faire sortir le produit liquide sous forme de gouttes par l'orifice non refermable. Ce type d'appareil est conçu pour délivrer une dose unique de produit (dose quotidienne formée d'une ou de plusieurs gouttes) et dès lors que l'utilisateur a extrait la dose prescrite du contenant, même si du produit reste dedans, il n'est pas possible de refermer l'orifice et donc le produit est susceptible de se détériorer au contact de l'air. Le coût de la dose prescrite est donc relativement cher puisqu'elle nécessite un contenant par dose.

Dans la deuxième catégorie on trouve les appareils qui se présentent sous la forme d'un flacon contenant plusieurs doses pour un traitement complet sur plusieurs jours. Cependant, le produit contenu dans le flacon est en contact avec l'air ambiant à chaque ouverture du flacon et il est donc exposé aux diverses pollutions véhiculées par l'air. De ce fait, les fabricants sont obligés d'ajouter au produit des agents conservateurs tels que des agents anti-bactériens, anti-oxydants... Toutefois, de tels agents sont susceptibles de générer des réactions avec les yeux des utilisateurs.

Compte tenu de ce qui précède, il existe donc un besoin d'un nouvel appareil de distribution de gouttes d'un produit liquide ou semi-liquide qui remédie à au moins un des inconvénients précités, notamment qui limite la pollution de l'appareil, et peut trouver de nombreuses applications incluant notamment le domaine ophtalmique.

On connait du document US 2013/0037574 un appareil de distribution de gouttes de produit liquide sous la forme de doses mais qui n'est toutefois pas satisfaisant.

La présente invention a ainsi pour objet un appareil de distribution de gouttes d'un produit liquide ou semi-liquide, caractérisé en ce qu'il comprend :
- un tube qui comprend, d'une part, une enveloppe externe déformable définissant un espace interne renfermant le produit et, d'autre part, une ouverture,
- un embout de génération de gouttes qui est disposé au niveau de l'ouverture et qui est apte, d'une part, à former et à expulser une goutte de produit de l'embout par suite d'une pression exercée sur l'enveloppe externe et qui tend à la déformer et à réduire le volume de l'espace interne et, d'autre part, à empêcher tout retour de produit dans l'embout lorsque la pression sur l'enveloppe cesse, l'embout de génération de gouttes comprenant un orifice à travers lequel une goutte se forme lors de la traversée dudit orifice par le produit par suite d'une pression exercée sur l'enveloppe externe, l'orifice étant délimité à sa périphérie par au moins une paroi déformable qui, en se déformant, autorise la formation d'une goutte,
- un bouchon de protection qui est fixé de manière amovible sur l'appareil afin de recouvrir l'embout de génération de gouttes en cas de non utilisation de l'appareil, le bouchon de protection comportant une face interne qui est orientée en vis-à-vis de l'embout de génération de gouttes lorsque le bouchon de protection est fixé sur l'appareil, la face interne du bouchon de protection étant pourvue d'au moins un élément en matériau absorbant destiné à absorber une goutte au moins partiellement formée à l'extérieur de l'orifice et qui n'est pas détachée de cet orifice, ledit au moins un élément en matériau absorbant étant distant de l'orifice.

Un tel appareil est capable de générer une seule goutte de produit ou consécutivement plusieurs gouttes de produit consécutives en appuyant simplement plusieurs fois ou de façon prolongée sur l'enveloppe externe déformable. Une fois qu'une goutte a été formée par l'embout de génération de gouttes à l'extérieur de celui-ci et, lorsque l'action de pression cesse sur l'enveloppe, la goutte ainsi formée ne peut plus rentrer dans l'embout (l'embout fait ainsi office de clapet qui s'ouvre et se ferme selon qu'une pression est exercée sur l'enveloppe ou non). Ainsi, l'air ambiant ne peut donc pas rentrer dans l'embout et le tube et contaminer le produit restant dans ce tube. Le produit restant est donc protégé de l'extérieur et peut se conserver dans le tube sur une période allant au-delà de plusieurs jours sans avoir besoin d'ajouter au produit différents agents tels que des agents conservateurs, anti-bactériens, anti-oxydants... Le produit est donc plus simple à fabriquer et il apparaît aux yeux des utilisateurs comme étant plus « sain » car comprenant moins de constituants.

La dose de produit fournie par l'appareil selon l'invention revient donc moins chère à distribuer qu'avec un appareil monodose de l'art antérieur. Le tube peut être un tube standard du marché.

Par ailleurs, l'agencement dudit au moins un élément en matériau absorbant à distance de l'orifice de l'embout permet audit au moins un élément en matériau absorbant de ne pas toucher ladite au moins une paroi déformable et notamment l'orifice (extrémité du clapet), et ainsi d'éviter de polluer cette zone.

On notera que la goutte partiellement formée à l'extérieur de l'embout (lorsque l'embout est dirigé vers le bas et que l'orifice se referme) n'a pas un poids suffisant pour compenser la tension superficielle qui la retient attachée à l'orifice. La goutte reste donc collée à l'orifice. L'agencement précité permet ainsi d'absorber, sans contact avec l'embout, le surplus de produit formé à l'extérieur de l'embout et adhérant à celui-ci, en évitant toute pollution de l'embout. On notera que ledit au moins un élément en matériau absorbant est disposé à une distance de l'orifice qui lui permet d'entrer en contact avec le surplus de produit adhérant à l'orifice ou à la paroi qui l'entoure. L'absorption se fait ainsi par capillarité du seul fait du contact dudit au moins un élément en matériau absorbant avec le surplus de produit externe.

Ledit au moins un élément en matériau absorbant est par exemple maintenu dans un logement prévu sur la face interne du bouchon. Le logement est par exemple réalisé à partir d'un ou de plusieurs éléments qui font saillie à partir de cette face et en éloignement de celle-ci (parallèlement à la hauteur ou dimension axiale du bouchon) , ce ou ces éléments formant une paroi externe délimitant le logement. La dimension transversale dudit au moins un élément en matériau absorbant (perpendiculairement à la hauteur du bouchon) est par exemple légèrement supérieure aux dimensions transversales du logement afin de pouvoir être introduit et maintenu en place dans ce dernier sans fixation mécanique ou chimique particulière. Il est ainsi possible de retirer aisément ledit au moins un élément en matériau absorbant pour le remplacer par un autre.

On notera que ladite au moins une paroi est dite déformable en ce sens qu'elle se déforme élastiquement sous l'action d'une contrainte (pression) et qu'elle revient à sa forme initiale non déformée lorsque la contrainte cesse. Cette propriété permet à ladite au moins une paroi de se déformer ainsi de manière répétée au cours du temps sans s'endommager.

Selon d'autres caractéristiques prises isolément ou en combinaison l'une avec l'autre :
- l'embout de génération de gouttes comprend des moyens d'obturation de l'orifice qui sont aptes à autoriser l'ouverture de l'orifice par suite d'une pression exercée sur l'enveloppe externe et, en l'absence d'une telle pression, à obturer l'orifice ;
- ladite au moins une paroi déformable est réalisée dans une matière souple ;
- ladite au moins une paroi souple est apte à se refermer :
   soit autour d'un organe rigide (ex : pointeau) en l'absence de pression sur l'enveloppe externe et à s'écarter de l'organe rigide suite à une pression exercée sur l'enveloppe externe (l'orifice est ouvert et l'organe rigide est dans l'orifice) ,
   soit sur l'orifice en l'absence de pression sur l'enveloppe externe (l'orifice est ainsi fermé lorsque ladite au moins une paroi est refermée);
- l'appareil est dépourvu de moyen de pompage du produit ;
- le tube comporte un fond qui est disposé dans une zone du tube opposée à la zone où est disposée l'ouverture, le tube étant rempli de produit par le fond ;
- l'appareil est apte à distribuer une goutte uniquement lorsque le tube est disposé avec l'embout de génération de gouttes dirigé vers le bas, ce qui convient particulièrement à certaines applications telles que la distribution de gouttes ophtalmiques;
- l'espace interne du tube est partiellement rempli de produit afin d'y ménager un volume libre prédéterminé qui est supérieur au volume de produit contenu dans la zone de l'appareil qui est indéformable, le volume libre prédéterminé étant occupé par un gaz;
- la face interne du bouchon de protection est configurée pour prendre appui sur ladite au moins une paroi déformable et empêcher sa déformation lorsque le bouchon de protection est fixé sur l'appareil ; lorsque la face interne du bouchon (au moins une partie de celle-ci) est disposée ou plaquée contre ladite au moins une paroi déformable (cette dernière ou ces dernières formant une ou des lèvres autour de l'orifice et qui, en se déformant sous l'action de la pression exercée sur l'enveloppe, ouvrent ou élargissent l'orifice), elle empêche toute déformation de ladite au moins une paroi déformable qui tendrait à ouvrir l'orifice (si l'orifice est fermé au repos) ou à l'élargir (s'il est déjà ouvert au repos et repose par exemple sur un pointeau) ;cette disposition permet de rendre étanche de manière simple et fiable, sans engendrer de pollution de l'embout, l'appareil équipé du bouchon ; la face interne du bouchon de protection est par exemple configurée dans une zone qui entoure ledit au moins un élément en matériau absorbant équipant une portion de la face interne du bouchon ; la forme ou configuration de la face interne du bouchon (ou d'une partie de celle-ci) qui permet de maintenir ou contraindre ladite au moins une paroi déformable dans sa position non déformée ou de repos peut revêtir différents aspects et par exemple prendre l'aspect d'une ou de plusieurs extensions d'une partie de cette face interne en éloignement de celle-ci (on notera que cette ou ces extensions peuvent correspondre à l'élément ou aux éléments formant la paroi du logement recevant ledit au moins un élément en matériau absorbant) ; l'extension peut être axiale (perpendiculaire à la partie de la face interne du bouchon qui est perpendiculaire à l'axe du tube lorsque le bouchon est sur le tube) ou adopter d'autres orientations (latérales ou radiales) ; on notera que la ou les extensions précitées sont généralement disposées autour dudit au moins un élément en matériau absorbant ;
- le bouchon de protection est percé d'un trou ouvert sur l'extérieur de l'appareil ; ceci permet d'aérer la zone interne au bouchon et situé derrière le trou ;
- ledit au moins un élément en matériau absorbant est disposé entre le trou traversant et l'embout de génération de gouttes de manière à permettre l'évaporation du produit absorbé par ledit au moins un élément en matériau absorbant ; ledit au moins un élément en matériau absorbant communique ainsi avec l'extérieur de l'appareil équipé de son bouchon afin de réaliser l'évaporation vers l'extérieur du produit absorbé ;
- ledit au moins un élément en matériau absorbant est disposé en vis-à-vis de l'orifice de l'embout;
- l'appareil tel que brièvement exposé ci-dessus est appliqué à la distribution de gouttes ophtalmiques.

L'invention a également pour objet un procédé de distribution de gouttes d'un produit liquide ou semi-liquide à partir d'un appareil, caractérisé en ce que l'appareil comprend :
- un tube comprenant, d'une part, une enveloppe externe déformable définissant un espace interne renfermant le produit et, d'autre part, une ouverture,
- un embout de génération de gouttes qui est disposé au niveau de l'ouverture et comprend un orifice qui est délimité à sa périphérie par au moins une paroi déformable,
   lorsque le tube est disposé avec l'embout de génération de gouttes dirigé vers le bas le procédé comprenant au moins les étapes i) et ii) suivantes :
   i) application d'une pression externe sur l'enveloppe externe déformable de l'appareil en vue de la déformer et de réduire le volume de l'espace interne afin, d'une part, de former au moins une goutte de produit par suite de la déformation de ladite au moins une paroi déformable et de la traversée de l'orifice de l'embout par le produit et, d'autre part, d'expulser ladite au moins une goutte ainsi formée hors de l'embout,
   ii) cessation de l'application d'une pression externe sur l'enveloppe externe déformable de l'appareil, le produit situé à l'extérieur de l'orifice de l'embout étant empêché de revenir dans l'embout par suite de la déformation inverse de ladite au moins une paroi déformable et de la refermeture au moins partielle de l'orifice, le produit situé à l'extérieur de l'orifice étant ainsi suspendu à l'orifice,
   iii) mise en place d'un bouchon de protection sur l'appareil afin de recouvrir l'embout, le bouchon comportant au moins un élément en matériau absorbant qui est distant de l'orifice lorsque le bouchon est fixé sur l'appareil et qui, par contact avec le produit suspendu à l'orifice à l'extérieur de celui-ci, absorbe ledit produit.

Un surplus de produit non distribué peut être présent une fois que la pression sur l'enveloppe cesse, que ladite au moins une paroi déformable est revenue à sa position initiale (non déformée) et que l'orifice s'est refermé au moins en partie (c'est-à-dire que la taille de l'orifice est revenue à sa taille initiale, à savoir que l'orifice est partiellement ou complètement refermé selon le cas), interdisant ainsi tout retour de produit à l'intérieur de l'embout et toute nouvelle sortie de produit. La mise en place d'un bouchon de protection comportant, maintenu à l'intérieur dudit bouchon, au moins un élément en matériau absorbant permet d'absorber un surplus de produit restant à l'extérieur de l'orifice. Ceci permet d'éviter de laisser du produit sur l'orifice (ou ladite au moins une paroi déformable) qui serait pollué par l'air environnant et contaminerait ainsi également ladite au moins une paroi déformable entourant l'orifice. La mise en place du bouchon peut se faire avec l'embout dirigé vers le bas ou vers le haut. Le procédé comporte les mêmes avantages que l'appareil exposé ci-dessus.

Selon d'autres caractéristiques prises isolément ou en combinaison l'une avec l'autre :
- l'étape i) est répétée au cours du temps afin de distribuer plusieurs gouttes de produit ;
- l'ensemble des étapes i) à iii) sont répétées après au moins une heure ;
- l'ensemble des étapes i) à iii) sont répétées après au moins un jour ;
- le procédé est appliqué à la distribution de gouttes ophtalmiques.

On notera que l'étape i) ci-dessus peut être répétée plusieurs fois avant que l'étape ii) n'intervienne et qu'un surplus de produit non souhaité apparaisse. Dans un tel cas de figure, une fois l'étape i) effectuée, l'utilisateur relâche la pression sur l'enveloppe externe du tube avant d'appuyer de nouveau pour distribuer une autre goutte de produit et ainsi de suite jusqu'à ce que le nombre souhaité de gouttes ait été expulsé et détaché de l'embout. L'étape suivante ii) peut alors survenir avant que le bouchon ne soit remis en place, voire ne pas survenir et le bouchon est malgré tout remis en place.

Toutes les caractéristiques précédentes exposées ci-dessus en relation avec l'appareil peuvent également être reprises ici en relation avec le procédé.

D'autres caractéristiques et avantages apparaitront au cours de la description qui va suivre, donnée uniquement à titre d'exemple non limitatif et faite en référence aux dessins annexés, sur lesquels :
- la figure 1 est une vue schématique générale d'un appareil de distribution de gouttes selon un premier mode de réalisation de l'invention ;
- la figure 2 est une vue de l'appareil de la figure 1 en position retournée en vue de son utilisation ;
- la figure 3 est une vue analogue de l'appareil de la figure 2 en cours d'utilisation ;
- la figure 4 est une vue schématique partielle d'un appareil de distribution de gouttes selon un deuxième mode de réalisation de l'invention ;
- la figure 5 est une vue schématique partielle d'un appareil de distribution de gouttes selon un troisième mode de réalisation de l'invention.

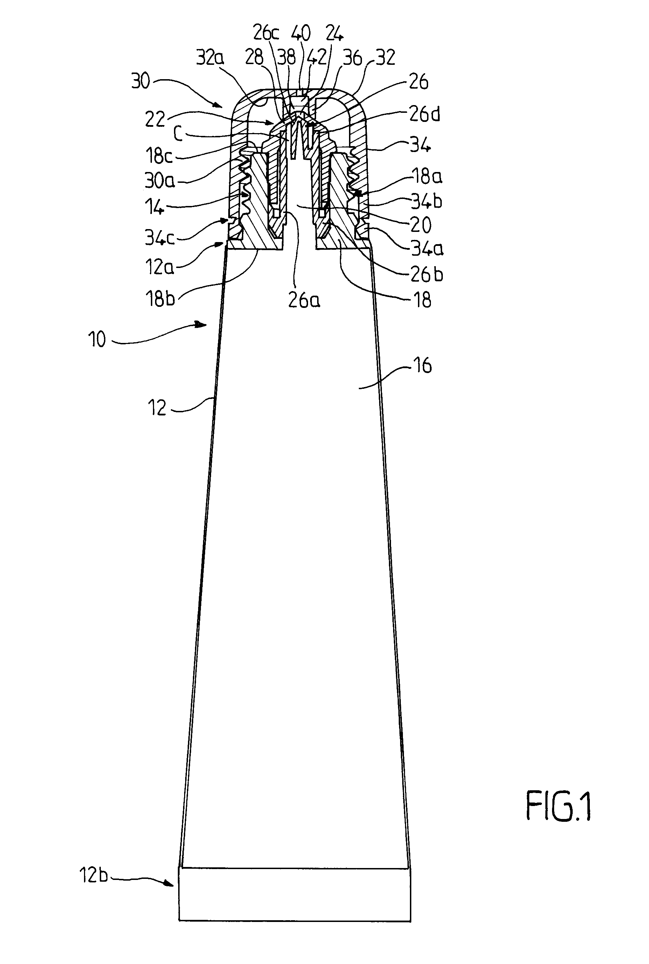

Comme représenté à la figure 1 et désigné par la référence générale notée 10, un appareil de distribution de gouttes d'un produit liquide ou semi-liquide selon un premier mode de réalisation de l'invention comprend un contenant 12 en forme de tube souple sur lequel est monté un embout de génération de gouttes 14. Dans ce mode de réalisation, le produit est liquide et il s'agit par exemple d'un produit ophtalmologique. Alternativement, le produit peut être par exemple une crème très fluide telle qu'un lait, voire un gel.

Sur la figure 1 le tube est disposé verticalement en position de rangement, avec l'embout positionné en haut du tube. Le tube est un tube conventionnel du marché.

Plus particulièrement, le tube 12 se présente sous la forme d'une enveloppe externe déformable, par exemple réalisée en polyéthylène ou en polypropylène, et définissant un espace interne 16 qui renferme le produit à distribuer (non représenté sur la figure 1).

Le tube 12 comporte deux extrémités opposées, à savoir une extrémité supérieure 12a sur la figure 1 qui est pourvue d'une première ouverture au niveau de laquelle est fixé l'embout 14 et une extrémité inférieure 12b qui forme un fond de tube. Lors de la fabrication du tube le fond de tube est laissé ouvert (seconde ouverture) pour le remplissage du tube en produit. Une fois le produit introduit dans l'espace interne 16 du tube, le fond est refermé, par exemple en réalisant un pincement soudé des bords de l'enveloppe externe délimitant la seconde ouverture.

L'embout de génération de gouttes 14 comprend une partie 18 formant col qui est fixée au niveau des bords de la première ouverture (l'embout est par exemple monté par un ajustement en force dans l'ouverture, mais il peut alternativement être encliqueté, vissé...) qui définit, dans sa portion centrale, un passage 20 pour le produit à distribuer. La partie formant col 18 possède sur sa surface extérieure un filetage externe 18a et a une forme générale sensiblement cylindrique qui s'étend axialement depuis une base élargie 18b fixée à l'enveloppe du tube jusqu'à un sommet 18c. Le passage central 20 s'étend axialement depuis la base jusqu'au sommet du col 18.

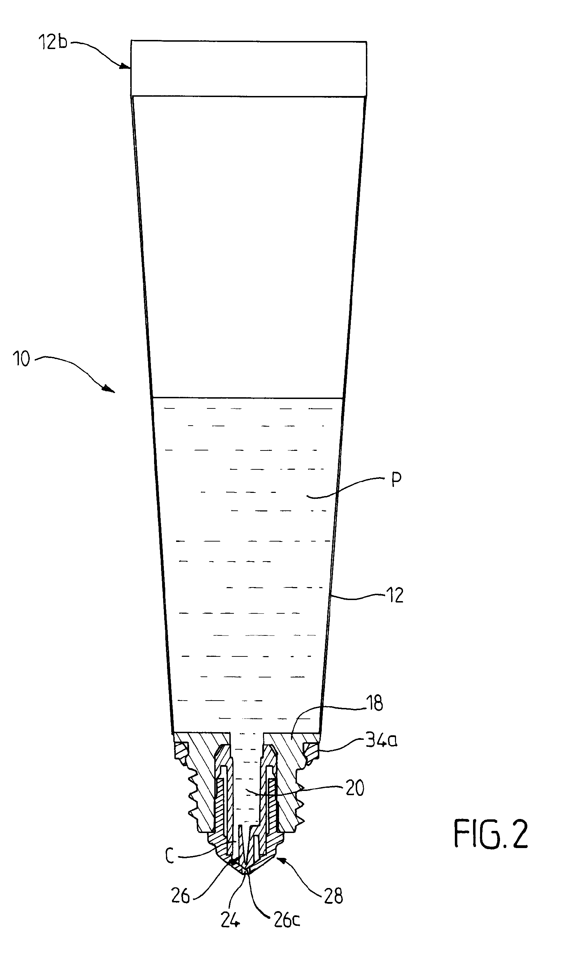

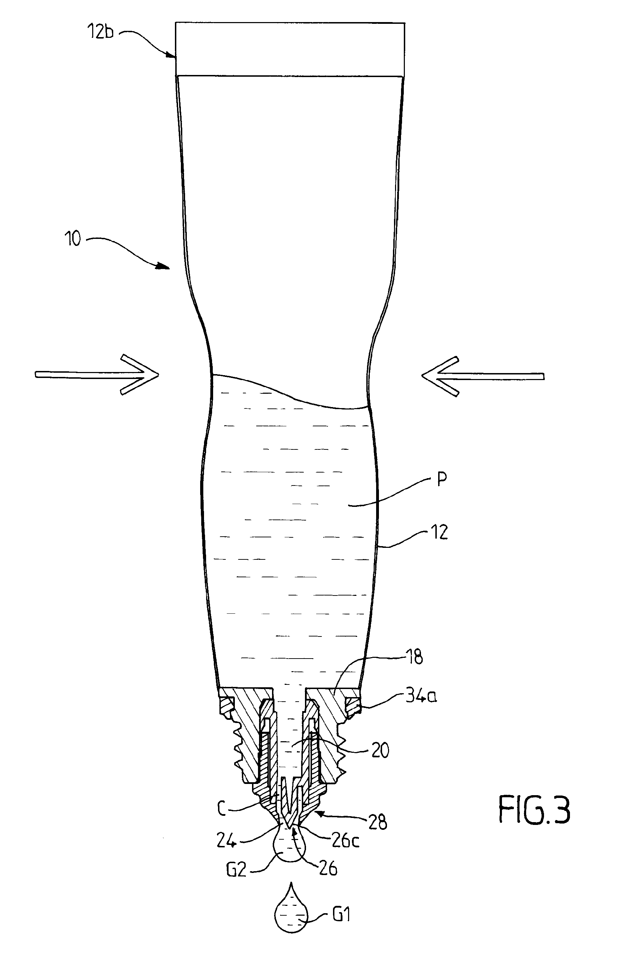

L'embout 14 comprend également un système de clapet 22 disposé en partie à l'intérieur du passage 20 et qui est apte, d'une part, à former une goutte de produit et à l'expulser hors du système de clapet (lorsque le tube est retourné en position d'utilisation comme sur les figures 2 et 3) par suite d'une pression extérieure exercée sur l'enveloppe externe et, d'autre part, à empêcher tout retour de produit dans le système de clapet lorsque la pression extérieure sur l'enveloppe cesse.

Le système de clapet 22 comprend de manière générale deux éléments fonctionnels:
- un orifice 24 à travers lequel une goutte se forme lors de la traversée de l'orifice par du produit par suite d'une pression exercée sur l'enveloppe externe,
- des moyens d'obturation de l'orifice 24 qui sont aptes à autoriser l'ouverture de l'orifice par suite d'une pression exercée sur l'enveloppe externe et, en l'absence d'une telle pression, à obturer l'orifice.

Les moyens d'obturation de l'orifice comprennent deux éléments principaux :
- un organe rigide (ex : pointeau) 26 qui s'étend dans le passage 20,
- une pièce ou paroi souple 28 (élastiquement déformable telle qu'une lèvre qui se déforme sous la pression ; ex : paroi en élastomère) disposée autour de l'organe rigide 26 et qui est en appui, d'une part, sur la surface extérieure de l'organe rigide 26 et, d'autre part, sur le sommet 18c du col 18 et sur la surface interne de ce dernier.

L'orifice 24 est pratiqué dans la portion centrale (disposée suivant l'axe longitudinal du passage 20) de paroi souple 28.

L'organe rigide 26 comprend une première partie 26a de forme sensiblement cylindrique qui est montée à l'intérieur du passage 20 et est en appui sur la surface interne du col 18 par son extrémité inférieure 26b.

L'organe rigide comprend également une deuxième partie 26c formant pointeau fixée à l'extrémité supérieure 26d de l'organe et en partie à l'intérieur de ce dernier. La deuxième partie 26c s'étend en partie à l'intérieur de la première partie 26a et ménage avec la surface interne de celle-ci un canal annulaire c pour l'écoulement du produit depuis le passage 20 jusqu'à la paroi souple 28. Les deux parties 26a et 26c de l'organe rigide sont dans une position fixe l'une par rapport à l'autre.

Dans la position de repos (non utilisation) de la figure 1, la paroi souple 28 s'étend par-dessus la deuxième partie 26c de manière à la recouvrir, sauf à l'aplomb de l'orifice 24. En l'absence d'une pression exercée sur l'enveloppe externe du tube (figure 1) les bords de la paroi souple 28 qui délimitent l'orifice 24 (ces bords forment en quelque sorte des lèvres déformables) viennent en contact avec l'extrémité sensiblement conique de la deuxième partie 26c. L'élasticité du matériau constituant la paroi souple 28 permet d'assurer un contact étanche. L'orifice 24, quant à lui, reste ouvert et la pointe de l'extrémité sensiblement conique de la deuxième partie 26c y pénètre en partie. Le système de clapet décrit ci-dessus est décrit plus en détails dans le brevet français n° 2 916 657.

Comme représenté sur la figure 1, l'appareil 10 comprend un bouchon de protection 30 qui est fixé de manière amovible sur l'appareil afin de recouvrir l'embout de génération de gouttes 14 en cas de non utilisation de l'appareil. Dans le présent mode de réalisation, le bouchon 30 comporte sur sa surface interne un filetage 30a complémentaire du filetage 18a qui, par coopération, vont permettre le dévissage et le vissage ultérieurs du bouchon par rapport à l'embout.

Le bouchon de protection 30 comporte une partie sensiblement plane 32 (ici horizontale) formant un fond et une partie formant jupe 34 qui s'étend perpendiculairement (ici suivant un axe vertical) à partir du fond 32. Le filetage 30a complémentaire du filetage 18a est aménagé sur la face interne de la jupe 34.

Le fond 32 possède une face interne 32a qui est orientée vers l'intérieur du bouchon et, plus particulièrement, en vis-à-vis de l'embout 14 lorsque le bouchon 30 est fixé sur l'appareil (figure 1). Le fond 32 comporte dans sa zone centrale une paroi cylindrique ou collerette 36 qui s'étend parallèlement à la jupe 34 (direction axiale) en éloignement de la face interne 32a. La paroi cylindrique 36 délimite une cavité intérieure centrale 38 disposée en regard de l'orifice 24. Le fond 32 est percé en son centre, à l'aplomb de la cavité centrale ou logement 38, d'un trou traversant 40. Un élément en matériau absorbant 42 (par exemple réalisé en fibre, en feutre, en mousse, par exemple en mousse de polyéthylène......) est logé et maintenu au fond de la cavité 38 de manière à être en contact avec le trou 40 (ou très près de celui-ci) et agencé en vis-à-vis de l'orifice 24. Comme illustré sur la figure 1, l'élément en matériau absorbant 42 est disposé entre le trou 40 et l'embout 14 et à distance de la paroi souple 28 et donc de l'orifice 24 pour éviter tout risque de pollution de ceux-ci. L'élément 42 prend par exemple la forme d'un disque.

Comme représenté sur la figure 1, lorsque le bouchon 30 est monté fixé sur l'appareil, l'extrémité libre de la paroi 36 solidaire du fond 32 est en appui ou plaquée contre les bords de la paroi souple 28 qui délimitent l'orifice 24. Cette disposition assure ainsi le verrouillage en position de la paroi souple 28 maintenue contre la deuxième partie 26c de l'organe rigide 26. L'étanchéité de l'appareil ainsi équipé est donc assurée.

La jupe 34 du bouchon est formée de deux portions : une première portion inférieure 34a fixée (par exemple par encliquetage) au niveau d'un épaulement formé sur la surface extérieure du col 18 de l'embout 14, en dessous du filetage 18a et une deuxième portion supérieure 34b formant la jupe et solidaire du fond 32. Les deux portions 34a et 34b sont reliées l'une à l'autre par une zone amincie 34c constituant une zone fragilisée mécaniquement qui facilite la rupture entre les deux portions par dévissage de la deuxième portion supérieure 34b et, de ce fait, la séparation de celles-ci. La première portion 34a constitue une bague d'inviolabilité dont la présence sur l'appareil garantit que l'appareil n'a pas encore été utilisé, lorsque les deux portions 34a, 34b de la jupe sont toujours reliées entre elles.

On notera que l'appareil 10 ne comporte pas de moyen de pompage, notamment pas de moyen de pompage comprenant un poussoir monté sur une pompe doseuse, permettant de faire monter le produit jusqu'en haut de l'appareil en vue de sa distribution. Avec un tel moyen de pompage, l'utilisateur ne gère pas la dose de produit distribuée car celle-ci est imposée par la pompe. L'appareil 10 selon le mode de réalisation de l'invention est donc plus simple de conception qu'un appareil qui nécessiterait un tel moyen de pompage.

L'appareil 10 ne peut ainsi être utilisé qu'en position renversée tel que représenté sur les figures 2 et 3.

A la première utilisation de l'appareil, le bouchon de protection 30 est retiré (par dévissage de la jupe 34) et l'appareil est renversé comme illustré sur la figure 2. Le produit liquide P occupe ainsi la partie de l'appareil équipée de l'embout 14 et pénètre dans le passage 20 et dans le canal c, mais est retenu par la paroi souple 28 qui est toujours refermée autour de l'organe rigide 26.

Pour distribuer une goutte de produit P l'utilisateur tient le tube entre les doigts d'une main et presse sur l'enveloppe externe déformable du tube 12 comme indiqué par les flèches sur la figure 3. En déformant ainsi l'enveloppe, le volume de l'espace interne 16 renfermant le produit se réduit, ce qui génère une montée en pression du produit liquide dans l'enveloppe. La pression du produit liquide à l'intérieur du tube est ainsi proportionnelle à la force d'appui de l'utilisateur sur le tube. La force exercée par l'utilisateur est par exemple comprise entre 200 et 500g.

Le produit liquide sous pression pousse sur la face interne de la paroi souple 28, écartant ainsi vers l'extérieur (radialement) les bords de la paroi qui délimitent l'orifice 24. L'orifice 24 s'écarte de la deuxième partie 26c de l'organe rigide 26 et s'élargit, laissant ainsi s'écouler le produit liquide à travers l'orifice (sortie cylindrique). En pressant lentement, de façon constante avec une pression raisonnable sur le tube, les phénomènes d'adhérence des matières en contact avec le produit (tensions superficielles) permettent de maintenir le produit en suspension à l'extrémité du système de clapet et ainsi de former une goutte de produit. Cette goutte chute lorsque son poids est supérieur à la résistance générée par les tensions superficielles qui retiennent la goutte.

Sur la figure 3, une première goutte G1 a été formée et expulsée hors de l'embout et une deuxième goutte G2 en cours de formation reste « accrochée » à l'extrémité du système de clapet. On notera que le volume de la goutte est défini par plusieurs paramètres que sont les formes, les dimensions, les états de surface, les matières du système de clapet ainsi que la constitution même du produit (viscosité,...).

Une goutte de produit ainsi formée correspond à une dose de produit. L'utilisateur gère sa dose puisqu'il génère lui-même les gouttes tandis que dans un appareil muni de moyen de pompage c'est la pompe qui gère la dose mais elle ne gère pas des gouttes.

Lorsque l'utilisateur cesse d'appuyer sur le tube, la pression du produit liquide dans ce dernier diminue et, grâce à l'élasticité de la matière constitutive de la paroi souple 28, la paroi se referme autour de l'organe rigide 26 et reprend sa forme initiale, de même que l'orifice 24 (figures 1 et 2). Le canal c est ainsi refermé empêchant tout passage du produit hors de l'embout.

Lorsque la paroi souple 28 reprend sa forme initiale, les bords de la paroi qui délimitent l'orifice 24 (lèvres déformables) se resserrent et séparent ainsi la goutte G2 en cours de formation du produit resté à l'intérieur du système de clapet. La goutte G2 partiellement formée reste ainsi en suspension sur l'extrémité extérieure du système de clapet en position refermée. De ce fait, la goutte n'est pas réaspirée à l'intérieur du système de clapet lorsque la pression sur le tube cesse. En effet, le poids de la goutte partielle G2 est inférieur à la tension superficielle qui la maintient attachée (en contact) à l'orifice 24 et les bords de la paroi déformable. La goutte partielle G2 est ainsi suspendue à l'orifice. Cette fonction anti-retour de produit (anti-pollution) est avantageuse puisqu'elle permet d'éviter d'ajouter des agents conservateurs (agents anti-oxydants, anti-bactériens...) au produit à distribuer, ou en tout cas de limiter l'ajout de tels agents. Il est donc possible d'utiliser à nouveau l'appareil (appareil multi-doses) pour distribuer une ou plusieurs gouttes de produit (selon la dose souhaitée), immédiatement après ou bien ultérieurement dans la même journée, au bout d'une ou de plusieurs heures, voire un autre jour et, par exemple, de manière répétée au cours du temps sur une période prédéterminée.

On notera que pour générer plusieurs gouttes consécutivement on peut, soit alterner les étapes d'appui et de relâchement sur le tube, soit appuyer de manière prolongée sur celui-ci.

Si la goutte G2 illustrée sur la figure 3 n'est pas destinée à être utilisée, l'utilisateur retourne l'appareil pour le placer dans la position de la figure 1 et revisse le bouchon 30. Alternativement, le bouchon peut être revissé alors que l'appareil a la tête en bas.

L'élément en matériau absorbant 42 du bouchon est éloigné de l'orifice 24 à une distance telle qu'il vient en contact avec la goutte G2 adhérant à l'orifice et, par capillarité, absorbe cette dernière afin de protéger l'extrémité libre du système de clapet. Cette disposition permet de détacher rapidement de l'extrémité de l'embout générateur de gouttes la goutte qui a pu être polluée par l'air ambiant. On évite ainsi une trop grande prolifération des bactéries sur l'appareil de distribution. On évite également de ne pas souiller l'appareil lors de la remise en place du bouchon. Le trou d'évacuation à l'air libre 40 dans le fond du bouchon permet d'évaporer la goutte de produit qui a été absorbée par l'élément en matériau absorbant 42. Le bouchon verrouille la déformation de la paroi souple 28 (lèvres souples déformables) et rend donc le système de clapet (et l'embout) complètement étanche : aucune goutte ne peut se former (même lorsque l'appareil est retourné comme sur la figure 3).

On notera par ailleurs que, lors du remplissage du tube, le produit est introduit à l'intérieur de l'espace interne 16 du tube en laissant un volume libre prédéterminé inoccupé par le produit. Ce volume libre prédéterminé est supérieur au volume de produit contenu dans la zone de l'appareil qui est indéformable. Le volume prédéterminé à laisser libre de produit dépend du volume de la zone indéformable. Cette zone correspond à la zone du tube (généralement située au niveau de l'extrémité supérieure 12a du tube et en dessous la base 18b du col 18) dans laquelle du produit résiduel reste piégé en fin d'utilisation de l'appareil en raison de l'aspect indéformable de l'appareil dans cette zone. Le volume libre prédéterminé est ainsi occupé par un gaz tel que l'air (voire un gaz inerte tel que l'azote) qui, sous l'effet de la pression exercée par l'utilisateur sur l'enveloppe externe du tube, chasse le produit accumulé dans cette zone vers le passage 20. Cette disposition améliore le taux de restitution de l'appareil. A titre d'exemple, on laisse un volume d'air prédéterminé d'environ un tiers du volume de l'espace interne 16 du tube.

Le système de clapet de l'embout peut alternativement être remplacé par une paroi souple déformable dans laquelle est pratiquée une fente (orifice) et qui s'ouvre (en position retournée) sous l'effet de la pression extérieure exercée sur l'enveloppe externe du tube. Lorsque la pression cesse, la fente se referme sur elle-même et sépare la goutte en formation du produit resté dans le passage interne de l'embout. L'embout ne comporte par d'organe formant pointeau comme dans le mode de réalisation des figures 1 à 3. Tout ce qui a été dit ci-dessus à propos du bouchon de protection et de ses fonctionnalités (présence d'au moins un élément en matériau absorbant distant de l'orifice et/ou bouchon en appui par au moins une partie de sa face interne contre l'embout (contre la paroi entourant l'orifice) et/ou trou traversant dans la paroi du bouchon) s'applique également à cette solution alternative qui peut donc comporter également un tel bouchon.

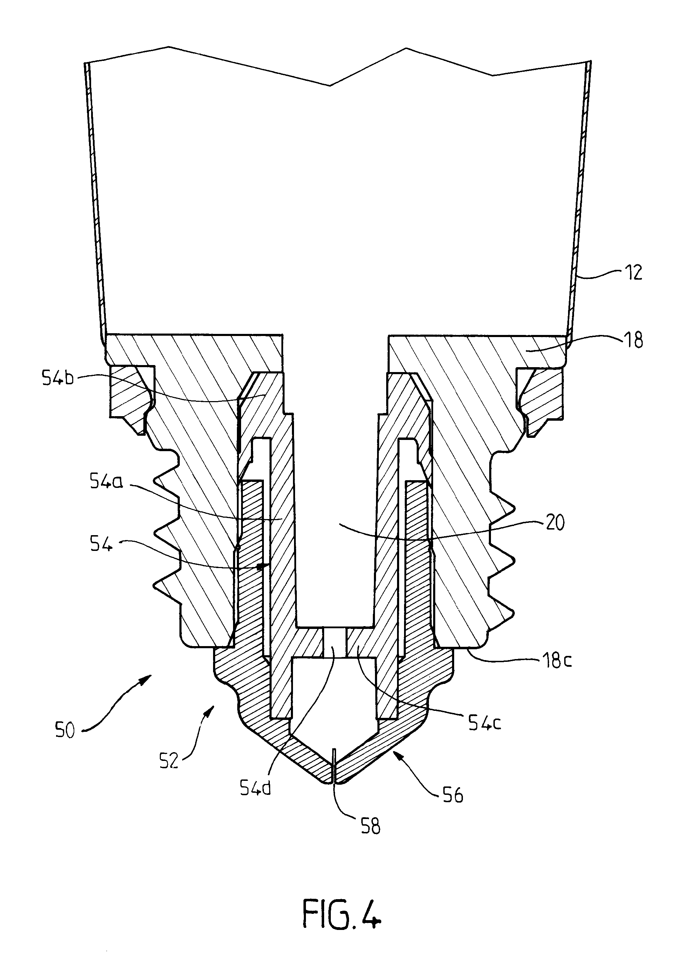

Un deuxième mode de réalisation d'un appareil de distribution de gouttes 50 est partiellement illustré sur la figure 4 en position retournée et les éléments communs à ceux du premier mode conservent les mêmes références. Seul l'embout diffère entre les deux modes. L'appareil 50 comprend un embout de génération de gouttes 52 qui comprend le col 18 et un organe rigide 54 qui est monté à l'intérieur du passage 20. L'organe rigide comprend une première partie 54a de forme sensiblement cylindrique qui est montée à l'intérieur du passage 20 et est en appui sur la surface interne du col 18 par son extrémité 54b qui est une extrémité inférieure en position de repos de l'appareil (l'extrémité 54b est représentée en haut sur la figure car l'appareil est en position retournée d'utilisation). La première partie 54a s'étend axialement dans le passage 20 au-delà du col 18. Une cloison transversale 54c percée d'un trou traversant 54d est aménagée à l'intérieur de la première partie 54a. L'embout 52 comprend également une pièce ou paroi souple 56 disposée autour de l'organe rigide 54 et qui est en appui, d'une part, sur la surface extérieure de l'organe rigide 54 et, d'autre part, sur le sommet 18c du col 18 et sur la surface interne de ce dernier. La pièce souple 56 comporte une fente 58 qui, dans la position de la figure 4 (l'utilisateur n'appuie pas sur l'enveloppe externe déformable du tube 12 comme sur la figure 3), est fermée. La fente 58 s'ouvre lorsque l'utilisateur presse le tube (comme sur la figure 3) sous l'effet de la mise en pression du produit qui traverse le trou 54d, s'accumule en aval de ce dernier et force ainsi par son poids l'ouverture de la fente et la génération d'une goutte.

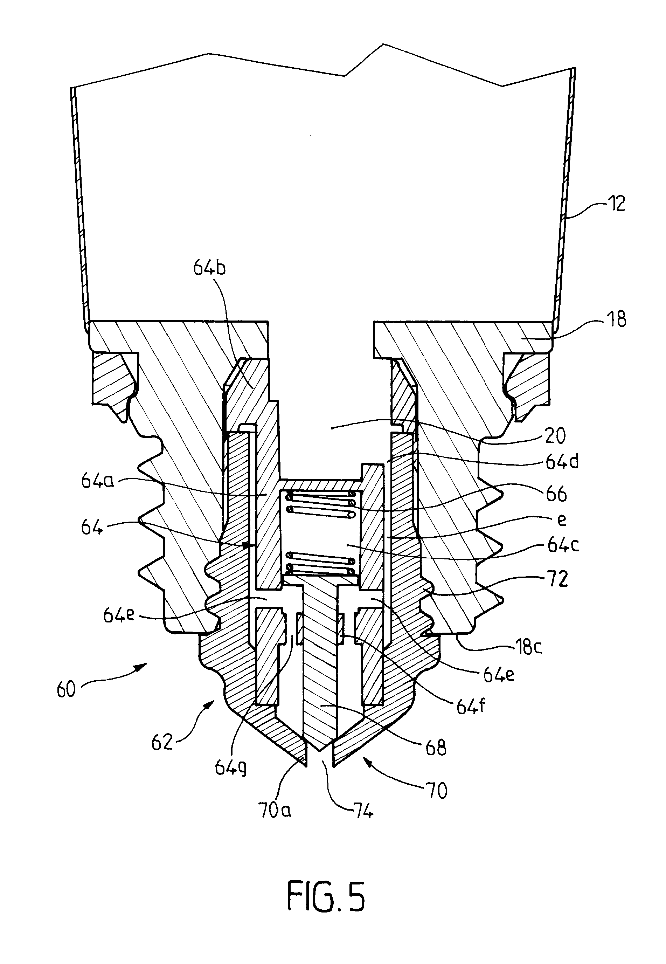

Un troisième mode de réalisation d'un appareil de distribution de gouttes 60 est partiellement illustré à la figure 5 en position retournée et les éléments communs à ceux du premier mode conservent les mêmes références. Seul l'embout 62 diffère de l'embout 14 du premier mode.

L'embout 62 comporte ainsi un organe rigide 64 qui est monté à l'intérieur du passage 20. L'organe rigide comprend une première partie 64a de forme sensiblement cylindrique qui est montée à l'intérieur du passage 20 et est en appui sur la surface interne du col 18 par son extrémité 64b qui est une extrémité inférieure en position de repos de l'appareil (l'extrémité 64b est représentée en haut sur la figure car l'appareil est en position retournée d'utilisation). La première partie 64a s'étend axialement dans le passage 20 au-delà du col 18.

La première partie 64a comprend, à l'intérieur du cylindre qu'elle forme, une chambre 64c logeant un moyen élastique tel qu'un ressort de compression 66. L'organe rigide 64 comprend également une pièce rigide mobile formant pointeau 68 qui est montée sur le moyen élastique 66 et est susceptible de coulisser axialement dans le passage 20.

L'embout 62 comprend également une pièce ou paroi 70 disposée autour de l'organe rigide 64 et qui est en appui, d'une part, sur la surface extérieure de l'organe rigide 64 et, d'autre part, sur le sommet 18c du col 18 et sur la surface interne de ce dernier. La pièce 70 est similaire à celle des figures 1 à 3. Toutefois, elle diffère de celle-ci notamment par le fait qu'elle est rigide et par la présence de moyens de liaison 72 entre la pièce 70 et la surface interne du col 18. Dans cet exemple, il s'agit de filetages complémentaires mais d'autres moyens alternatifs de liaison sont envisageables.

La pièce 70 est configurée à son extrémité par un bord périphérique 70a qui délimite un orifice central 74 toujours ouvert. Cet orifice 74 est obturé par la présence du pointeau mobile 68 en l'absence de pression externe sur le tube 12.

La première partie 64a est espacée de la surface interne de la pièce 70 à l'intérieur de laquelle elle est aménagée de manière à délimiter un espace cylindrique annulaire e (la première partie 64a est configurée pour laisser ouvert près de sa base une entrée 64d dans cet espace e). La première partie 64a est percée de trous radiaux 64e dans sa partie cylindrique pour une mise en communication de l'espace e avec l'espace interne à la première partie 64a. Une cloison transversale percée 64f est agencée à l'intérieur du cylindre que forme la première partie 64a afin de servir de guide au mouvement du pointeau 68 et, via les trous axiaux 64g, permettre le passage du produit.

Lorsque l'utilisateur presse le tube et met en pression le produit (comme sur la figure 3), le produit pénètre dans l'espace e, traverse les trous radiaux 64e, les trous axiaux 64g et s'accumule en amont de la pièce 70, entre celle-ci et la base du pointeau 72. La pression accumulée de produit provoque la remontée du pointeau dans la chambre 64c et la libération de l'orifice 74. Le produit est alors libre, sous l'action de son poids, de traverser l'orifice 74 (qui conserve sa forme initiale) en formant une goutte.

Selon une variante non représentée, le ressort 66 est supprimé, le pointeau 68 n'est plus mobile et seule la paroi 70 qui est souple s'élargit ou se resserre en fonction de la pression ou de l'absence de pression exercée sur le tube.

Selon une autre variante non représentée, le système de clapet de l'embout peut comporter, en plus d'une paroi souple déformable dont les bords délimitent un orifice fermé en position de repos, un organe rigide mobile qui, sous l'action du produit sous pression dans le tube et par un mécanisme approprié, force l'ouverture de l'orifice afin de permettre le passage du produit.

Toute autre structure d'embout qui est capable de former une goutte de produit liquide ou semi-liquide à l'extérieur de l'embout en appuyant sur le tube et d'empêcher le produit de rentrer dans l'embout lorsque la pression extérieure sur le tube est relâchée (comme c'est le cas des appareils décrits ci-dessus) convient également pour être fixée à un tube conventionnel à enveloppe externe déformable.

Les modes de réalisation des figures 4 et 5 et leurs variantes, ainsi qu'un appareil pourvu de toute autre structure d'embout telle que définie ci-dessus peuvent également comporter un bouchon de protection tel que défini plus haut (présence d'au moins un élément en matériau absorbant distant de l'orifice et/ou bouchon en appui par au moins une partie de sa face interne contre l'embout (contre la paroi entourant l'orifice) et/ou trou traversant dans la paroi du bouchon) et les avantages précités relatifs au bouchon s'appliquent également ici.

## Revendications

1. Appareil de distribution de gouttes d'un produit liquide ou semi-liquide, **caractérisé en ce qu'**il comprend :
- un tube (12) qui comprend, d'une part, une enveloppe externe déformable définissant un espace interne (16) renfermant le produit et, d'autre part, une ouverture,
- un embout de génération de gouttes (14) qui est disposé au niveau de l'ouverture et qui est apte, d'une part, à former et à expulser une goutte de produit (G1, G2) de l'embout par suite d'une pression exercée sur l'enveloppe externe et qui tend à la déformer et à réduire le volume de l'espace interne et, d'autre part, à empêcher tout retour de produit dans l'embout lorsque la pression sur l'enveloppe cesse, l'embout de génération de gouttes (14) comprenant un orifice (24) à travers lequel une goutte se forme lors de la traversée dudit orifice par le produit par suite d'une pression exercée sur l'enveloppe externe, l'orifice (24) étant délimité à sa périphérie par au moins une paroi déformable (28) qui, en se déformant, autorise la formation d'une goutte,
- un bouchon de protection (30) qui est fixé de manière amovible sur l'appareil afin de recouvrir l'embout de génération de gouttes (14) en cas de non utilisation de l'appareil, le bouchon de protection (30) comportant une face interne (32a) qui est orientée en vis-à-vis de l'embout de génération de gouttes (14) lorsque le bouchon de protection est fixé sur l'appareil, la face interne du bouchon de protection étant pourvue d'au moins un élément en matériau absorbant (42) destiné à absorber une goutte au moins partiellement formée à l'extérieur de l'orifice (24) et qui n'est pas détachée de cet orifice, ledit au moins un élément en matériau absorbant (42) étant distant de l'orifice (24).

2. Appareil selon la revendication 1, **caractérisé en ce que** l'embout de génération de gouttes (14) comprend des moyens d'obturation de l'orifice (24) qui sont aptes à autoriser l'ouverture de l'orifice par suite d'une pression exercée sur l'enveloppe externe et, en l'absence d'une telle pression, à obturer l'orifice.

3. Appareil selon la revendication 1 ou 2, **caractérisé en ce que** ladite au moins une paroi déformable (28) est réalisée dans une matière souple.

4. Appareil selon la revendication 3, **caractérisé en ce que** ladite au moins une paroi souple (28) est apte à se refermer :
- soit autour d'un organe rigide (26) en l'absence de pression sur l'enveloppe externe et à s'écarter de l'organe rigide suite à une pression exercée sur l'enveloppe externe,
- soit sur l'orifice (58) en l'absence de pression sur l'enveloppe externe.

5. Appareil selon l'une des revendications 1 à 4, **caractérisé en ce qu'**il est dépourvu de moyen de pompage du produit.

6. Appareil selon l'une des revendications 1 à 5, **caractérisé en ce qu'**il est apte à distribuer une goutte uniquement lorsque le tube est disposé avec l'embout de génération de gouttes dirigé vers le bas.

7. Appareil selon l'une des revendications 1 à 6, **caractérisé en ce que** la face interne (32a) du bouchon de protection est configurée pour prendre appui sur ladite au moins une paroi déformable (28) et empêcher sa déformation lorsque le bouchon de protection est fixé sur l'appareil.

8. Appareil selon l'une des revendications 1 à 7, **caractérisé en ce que** le bouchon de protection (30) est percé d'un trou (40) ouvert sur l'extérieur de l'appareil.

9. Appareil selon la revendication 8 **caractérisé en ce que** ledit au moins un élément en matériau absorbant (42) est disposé entre le trou traversant (40) et l'orifice (24) de manière à permettre l'évaporation du produit absorbé par ledit élément en matériau absorbant.

10. Appareil selon l'une des revendications 1 à 9, **caractérisé en ce que** ledit au moins un élément en matériau absorbant (42) est disposé en vis-à-vis de l'orifice (24) de l'embout.

11. Appareil selon la revendication 7 ou l'une des revendications 8 à 10 lorsque celles-ci dépendent de la revendication 7, **caractérisé en ce que** la face interne (32a) du bouchon de protection est ainsi configurée dans une zone qui entoure ledit au moins un élément en matériau absorbant (42).

12. Application de l'appareil selon l'une des revendications 1 à 11 à la distribution de gouttes ophtalmiques.

13. Procédé de distribution de gouttes d'un produit liquide ou semi-liquide à partir d'un appareil, **caractérisé en ce que** l'appareil comprend :
- un tube (12) comprenant, d'une part, une enveloppe externe déformable définissant un espace interne (16) renfermant le produit et, d'autre part, une ouverture,
- un embout de génération de gouttes (14) qui est disposé au niveau de l'ouverture et comprend un orifice (24) qui est délimité à sa périphérie par au moins une paroi déformable (28),
lorsque le tube est disposé avec l'embout de génération de gouttes dirigé vers le bas le procédé comprenant au moins les étapes i) et ii) suivantes :
i) application d'une pression externe sur l'enveloppe externe déformable de l'appareil en vue de la déformer et de réduire le volume de l'espace interne afin, d'une part, de former au moins une goutte de produit par suite de la déformation de ladite au moins une paroi déformable (28) et de la traversée de l'orifice (24) de l'embout par le produit et, d'autre part, d'expulser ladite au moins une goutte ainsi formée hors de l'embout,
ii) cessation de l'application d'une pression externe sur l'enveloppe externe déformable de l'appareil, le produit situé à l'extérieur de l'orifice (24) de l'embout étant empêché de revenir dans l'embout par suite de la déformation inverse de ladite au moins une paroi déformable (28) et de la refermeture au moins partielle de l'orifice (24), le produit situé à l'extérieur de l'orifice (24) étant ainsi suspendu à l'orifice (24),
iii) mise en place d'un bouchon de protection sur l'appareil afin de recouvrir l'embout, le bouchon comportant au moins un élément en matériau absorbant (42) qui est distant de l'orifice (24) lorsque le bouchon est fixé sur l'appareil et qui, par contact avec le produit suspendu à l'orifice (24) à l'extérieur de celui-ci, absorbe ledit produit.

14. Procédé selon la revendication 13, **caractérisé en ce que** l'étape i) est répétée au cours du temps afin de distribuer plusieurs gouttes de produit.

15. Procédé selon la revendication 13 ou 14, **caractérisé en ce qu'**il est appliqué à la distribution de gouttes ophtalmiques.
